# FASCICULE DE BREVET EUROPEEN

(11) **EP 1 119 564 B1**
(45) Date de publication et mention de la délivrance du brevet: **18.12.2002**
(21) Numéro de dépôt: 99970390.3
(22) Date de dépôt: 07.10.1999
(51) Int. Cl.: C07D 401/12, A61K 31/445

(54) **DERIVES D'ARYL- 4-FLUORO-4- (2-PYRIDIN-2-YL-ETHYLAMINO)-METHYL]-PIPERIDIN-1-YL -METHANONE COMME AGONISTES DU RECEPTEUR 5-HT1**
ARYL-4-FLUORO-4-(2-PYRIDIN-2-YL-ETHYLAMINO)-METHYL]-PIPERIDIN-1-YL-METHANONE DERIVATE ALS 5-HT1-REZEPTOR-AGONISTEN
ARYL- 4-FLUORO-4- (2-PYRIDIN-2-YL-ETHYLAMINO)-METHYL]-PIPERIDIN-1-YL -METHANONE DERIVATIVES AS 5-HT1 RECEPTOR ANTAGONISTS

(30) Priorité: 09.10.1998 FR 9812660
(43) Date de publication de la demande: 01.08.2001
(73) Titulaire: PIERRE FABRE MEDICAMENT, 92100 Boulogne (FR)
(72) Inventeur: VACHER, Bernard, F-81100 Castres (FR); BONNAUD, Bernard, F-81090 Lagarrigue (FR); KOEK, Wouter, F-81290 Viviers-les-Montagnes (FR)
(74) Mandataire: Ahner, Francis
(86) Numéro de dépôt international: FR9902401
(87) Numéro de publication internationale: WO00021953

(56) Documents cités:
- EP-A- 0 661 266
- WO-A-98/22459

## Description

La sérotonine (5-hydroxytryptamine, 5-HT) est un neurotransmetteur du système nerveux central qui exerce ses fonctions physiologiques multiples par l'interaction avec des récepteurs 5-HT spécifiques. Ces récepteurs 5-HT ont été regroupés en plusieurs classes principales. Parmi ces classes principales, la classe 5-HT₁ comprend des récepteurs caractérisés par une affinité élevée pour la sérotonine. La classe 5-HT₁ est elle-même divisée en sous-classe de récepteurs dont les caractéristiques pharmacologiques et les distributions régionales dans le système nerveux central sont distinctes.

Des études cliniques de composés ayant une activité agoniste pour les récepteurs sérotoninergiques du sous-type 5-HT_{1A} ont démontré que les agonistes 5-HT_{1A} étaient efficaces dans le traitement de l'anxiété (J. Clin. Psychiatry 1987, 48, 3S) et de la dépression (Int. J. Neuropsychopharmacology 1998, 1, 18). De plus, des études chez l'animal ont démontré que les agonistes 5-HT_{1A} possèdent des propriétés analgésiques (Behav. Brain Res. 1995, 73, 1/2, 69) et neuroprotectrices (Arch. Int. Pharmacodyn. 1995, 329, 347).
Compte tenu du potentiel thérapeutique étendu des composés doté d'une activité agoniste pour les récepteurs sérotoninergiques du sous-type 5-HT_{1A}, la découverte de composés nouveaux possédant une telle activité est d'un grand intérêt en clinique humaine.
La Buspirone, agoniste 5-HT_{1A} utilisé en clinique, présente une affinité comparable pour les récepteurs sérotoninergiques du sous-type 5-HT_{1A} et pour les récepteurs dopaminergiques de la sous-famille D₂. Les récepteurs dopaminergiques de la sous-famille D₂ étant impliqués dans le contrôle de la motricité, des fonctions cognitives et neuroendocriniennes (La Lettre du Pharmacologue 1997, 11, 3), les substances actives sur les récepteurs dopaminergiques de la sous-famille D₂, tel que la Buspirone, sont donc susceptibles d'occasionner des troubles neurologiques et / ou de la motricité et / ou neuroendocriniens (CNS Drug 1996, 5, 215). Une activité dopaminergique ou antidopaminergique associée à une activité agoniste 5-HT_{1A} ne constitue donc pas une combinaison de propriétés souhaitable pour un agent destiné aux traitements des désordres neurologiques sensibles à une activation sérotoninergique médiéé par les récepteurs 5-HT_{1A}.
Le Brevet WO 9822459-A1 décrit des dérivés de la pyridyn-2-yl-méthylamine de la formule générale 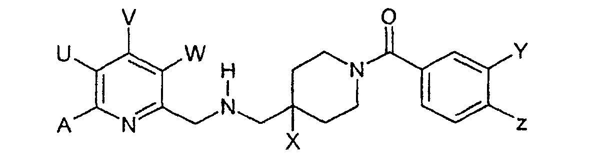 dans laquelle ;
A, U, V, W sont, entre autres, un atome d'hydrogène,
X représente un atome d'hydrogène ou de fluor,
Y est un atome de chlore ou un radical méthyl,
z est un atome d'hydrogène, un atome de fluor, un atome de chlore ou un radical méthyl.

Ces composés sont revendiqués comme agonistes 5-HT_{1A} utiles dans le traitement des troubles sensibles aux agonistes sérotoninergiques du sous-type 5-HT_{1A}.

La présente demande concerne des composés nouveaux répondant à la formule générale (1) 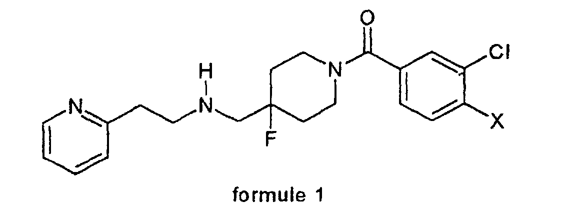 dans laquelle :
X est un atome d'hydrogène, de fluor ou de chlore,
   ainsi que les sels d'addition et les hydrates des sels d'addition des composés de formule générale (1) avec les acides minéraux ou les acides organiques pharmaceutiquement acceptables.

Les composés de formule (1) diffèrent de ceux décrits dans le brevet WO 9822459-A1 par la présence d'un groupe méthylène supplémentaire entre le noyau pyridinique et la fonction amine secondaire. L'incorporation du groupe méthylène supplémentaire se traduit par une diminution de la constante de dissociation dans l'eau (Ka) des composés de formule (1), d'un facteur dix en moyenne, par rapport à celle des composés décrits dans le brevet WO 9822459-A1. Un tel accroissement de la basicité modifie fortement les paramètres d'absorption, de distribution ainsi que les modes de transformations in vivo des composés de l'invention par rapport à ceux des composés revendiqués dans le brevet WO 9822459-A1.

L'intérêt des composés de l'invention réside à la fois dans leur sélectivité et leur activité agoniste 5-HT_{1A} in vivo très supérieure à celles de la Buspirone. A ce titre, les composés de l'invention, qui associent l'efficacité thérapeutique à une faible propension à manifester des effets secondaires indésirables d'origine dopaminergiques

D₂ tel que, par exemples, des désordres neurologiques et /ou de la motricité et / ou endocriniens, sont utiles dans le traitement des multiples pathologies impliquant des dysfonctionnements sérotoninergiques, en particulier l'anxiété, la dépression, la neurodégénerescence et la perception de la douleur.
La sélectivité est définie dans la présente demande comme étant le rapport des constantes d'affinité (Ki) D₂ / Ki (5-HT_{1A}) .
L'activité agoniste 5-HT_{1A} centrale des composés de l'invention et de la Buspirone a été évaluée, après administration orale chez le rat, par leur capacité à provoquer la rétraction de la lèvre inférieure de l'animal (LLR), un marqueur sensible et spécifique d'une activité agoniste 5-HT_{1A} centrale (Pharmacol. Biochem. Behav. 1989, 33, 821)
L'invention a aussi pour objet les compositions pharmaceutiques contenant à titre de principe actif au moins un dérivé de formule générale (1) ou un de ses sels ou hydrates de ses sels en combinaison avec un ou plusieurs excipients, adjuvants ou véhicules pharmaceutiquement acceptables. A titre d'exemple on peut citer les complexes d'inclusion, en particulier les complexes d'inclusion formés par les composés de l'invention avec les β-cyclodextrines.
Les compositions pharmaceutiques selon l'invention peuvent être des compositions administrables par voie orale, nasale, sublinguale, rectale ou parentérale. Il est généralement avantageux de formuler de telles compositions pharmaceutiques sous forme de dose unitaire. Chaque dose comprend alors une quantité prédéterminée du principe actif, associée au véhicule, excipients et / ou adjuvants appropriés, calculée pour obtenir un effet thérapeutique donné. A titre d'exemple de forme de dose unitaire administrable par voie orale on peut citer les comprimés, les gélules, les granules, les poudres et les solutions ou suspensions orales.
Les formulations appropriées pour la forme d'administration choisie sont connues et décrites par exemple dans le Remington, The Science and Practice of Pharmacy, 19^{ième} edition, 1995, Mack Publishing Compagny et peuvent donc être facilement préparées par l'homme de l'art.
Il est connu que la posologie varie d'un individu à l'autre, selon la nature et l'intensité de l'affection, la voie d'administration choisie, le poids, l'âge et le sexe du malade en conséquence les doses efficaces devront être déterminées en fonction de ces paramètres par le spécialiste en la matière. A titre indicatif, les doses efficaces pourraient s'échelonner entre 0.001 mg/Kg et 100 mg/Kg/jour.
Les composés de formule générale (1) peuvent exister sous plusieurs formes tautomères. De telles formes tautomères quoique non explicitement rapportées dans la présente demande pour simplifier la représentation graphique des formules sont néanmoins incluses dans le champ d'application de l'invention.
L'invention s'étend enfin au procédé de préparation des dérivés de formule générale (1).
Les composés de formule (1) ont été préparés selon la séquence réactionnelle indiquée dans le schéma A.

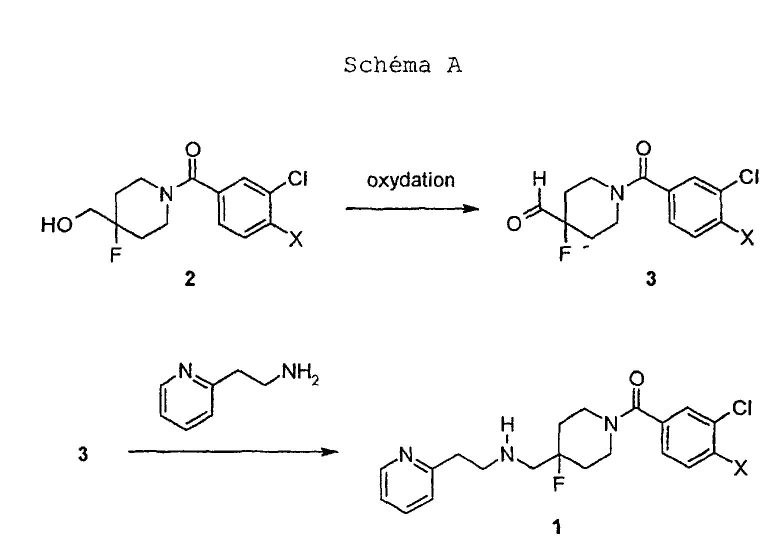

### Schéma A

Les composés de formule (2) ont été préparés selon une méthode analogue à celle utilisée pour la synthèse de la (4-fluoro-4-hydroxyméthyl-pipéridin-1-yl)-(3-chloro-4-fluoro-phényl)-méthanone et décrite dans le brevet WO 9822459-A1. L'oxydation du composé de formule (2), au moyen d'un dérivé activé du diméthyl sulfoxyde (DMSO) tel que par exemple du DMSO activé par le complexe trioxyde de sulfure-pyridine ou activé par le chlorure d'oxalyle, donne le 1-(3-chloro-4-x-benzoyl)-4-fluoro-pipéridine-4-carbaldéhyde de formule (3). Une réaction d'amination réductrice de l'aldéhyde de formule (3) au moyen de la 2-(2-Aminoéthyl)pyridine, disponible commercialement, conduit ensuite au composé de formule (1). Dans la réaction d'amination réductrice en question, l'aldéhyde (3) et la 2-(2-Aminoéthyl) pyridine sont mis en réaction dans le solvant approprié et le mélange est ensuite soumis à l'agent réducteur. L'agent réducteur en question peut être un hydrure de bore simple ou complexe tel que par exemple le borohydrure de sodium ou de potassium, le cyanoborohydrure de sodium ou le triacétoxyborohydrure de sodium.

Les exemples suivants illustrent l'invention et ne la limitent en aucune façon.

Dans les exemples ci-après :
(i) l'avancement des réactions est suivi par chromatographie couche mince (CCM) et par conséquent les temps de réaction ne sont mentionnés qu'à titre indicatif.
(ii) des formes cristallines différentes peuvent donner des points de fusions différents, les points de fusion rapportés dans la présente demande sont ceux des produits préparés selon la méthode décrite et ne sont pas corrigés.
(iii)la structure des produits obtenus selon l'invention est confirmée par les spectres de résonance magnétique nucléaire (RMN), infrarouge (IR) et l'analyse centésimale, la pureté des produits finaux est vérifiée par CCM.
(iv) les spectres RMN sont enregistrés dans le solvant indiqué. Les déplacements chimiques (δ) sont exprimés en partie par million (ppm) par rapport au tetraméthylsilane. La multiplicité des signaux est indiquée par : s, singulet ; d, doublet ; t, triplet ; q, quadruplet ; m, multiplet ; 1, large.
(v) les différents symboles des unités ont leur signification habituelle : mg (milligramme) ; g (gramme) ; ml (millilitre) ; °C (degré Celsius) ; mmole (millimole) ; nmole (nanomole) ; cm (centimètre).
(vi) Les abréviations ont la signification suivante : F (point de fusion) ; Eb (point d'ébullition).
(vii) Dans la présente application les pressions sont données en millibars ; par "température ambiante" on entend une température comprise entre 20°C et 25°C.

### Intermédiaire 1

### 1-(3-chloro-4-fluoro-benzoyl)-4-fluoro-pipéridine-4-carbaldéhyde (3; x = F)

Dans une solution de 1.5 g de 1-(3-chloro-4-fluoro-benzoyl)-4-fluoro-4-piperidine méthanol (5.18 mmoles), 2.16 ml de triethylamine (15.5 mmoles) et 15 ml de DMSO anhydre, sont ajouté en une seule fraction 2.48 g de complexe pyridine-SO₃ (15.5 mmoles). Après 3 heures d'agitation à température ambiante, la solution jaune obtenue est versée dans un mélange glace-eau et extraite deux fois par l'acétate d'éthyle. Les phases organiques sont lavées par une solution aqueuse d'acide citrique à 5% puis à l'eau salée, séchées sur MgSO₄, filtrées et le solvant est éliminé sous vide. On obtient 1.49 g (rendement quantitatif) d'une huile jaune qui est utilisée sans autre purification dans l'étape suivante.
IR ν (C=O) 1735 cm⁻¹
¹H RMN (CDCl₃) δ 1.50 - 2.05 (m, 4H) ; 3.34 (m, 2H) ; 3.70 (m, 1H) ; 4.59 (m, 1H) ; 7.18 (m, 1H) ; 7.31 (m, 1H) ; 7.50 (m, 1H) ; 9.77 (d, 1H)..

### Intermédiaire 2

### 1-(3,4-dichloro-benzoyl)-4-fluoro-pipéridine-4-carbaldéhyde (3; x = Cl)

Cet aldéhyde est obtenu par la même technique d'oxydation que celle utilisée pour la synthèse de l'intermédiaire 1 en remplaçant le 1-(3-chloro-4-fluoro-benzoyl)-4-fluoro-4-piperidine méthanol par le 1-(3,4-dichloro-benzoyl)-4-fluoro-4-piperidine méthanol. On obtient une huile jaune (rendement quantitatif) utilisée sans autre purification dans l'étape suivante.
IR ν (C=O) 1743 cm⁻¹.

### EXEMPLE 1 : (3-chloro-4-fluoro-phényl)-{4-fluoro-4-[(2-pyridin-2-yl-éthylamino)-méthyl]-piperidin-1-yl}-méthanone (1; x = F)

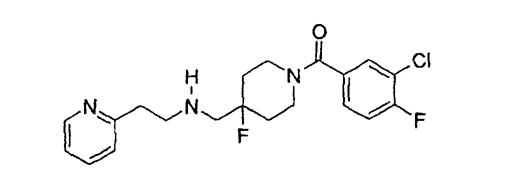

Une solution de 1.49 g de 1-(3-chloro-4-fluoro-benzoyl)-4-fiuoro-4-piperidine carboxaldehyde (5.18 mmoles), 0.70 g de (amino-2-éthyl)2-pyridine (5.70 mmoles) et 40 ml de toluène est maintenue au reflux sous agitation (en éliminant l'eau formée par un Dean-Stark) pendant 2 heures. Le toluène est éliminé par distillation sous pression réduite et le résidu obtenu est dissout dans 40 ml de méthanol anhydre. A cette solution, on ajoute par fractions, sous agitation et à température ambiante, 0.55 g de KBH₄ (10 mmoles), l'agitation est poursuivie pendant une nuit à température ambiante. Après élimination du méthanol sous vide, le résidu est extrait 2 fois par l'acétate d'éthyle, lavé à l'eau salée puis séché sur MgSO₄ et filtré. Le solvant est éliminé par distillation sous vide et le produit est purifié par chromatographie sur silice en utilisant le chlorure de méthylène à 5% de méthanol comme éluant. On obtient 1.43 g (72%) d'une huile jaune pâle. La salification par l'acide oxalique, effectuée dans un mélange éthanol-acétate d'éthyle, permet d'obtenir l'oxalate sous forme de cristaux blancs.
C₂₀H₂₂ClF₂N₃O, C₂H₂O₄ (483.91)
F : 172-174°C
¹H RMN (DMSO d₆) δ 1.73-1.99 (m, 4H) ; 3.12 (m, 3H) ; 3.24-3.32 (m, 5H) ; 3.47 (m, 1H) ; 4.30 (m, 1H) ; 7.28 (q, 1H) ; 7.32 (d, 1H) ; 7.46 (m, 1H) ; 7.52 (t, 1H) ; 7.68 (d, 1H) ; 7.76 (t, 1H) ; 8.50 (d, 1H).

### EXEMPLE 2 : (3,4-dichloro-phényl)-{4-fluoro-4-[(2-pyridin-2-yl-éthylamino)-méthyl]-pipéridin-1-yl}-méthanone (1; X = Cl)

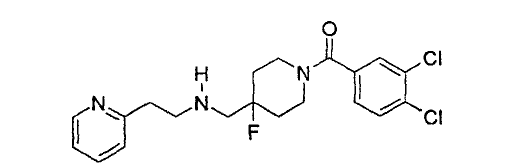

Ce composé est obtenu sous forme d'oxalate suivant le même mode opératoire que celui utilisé pour la préparation du (3-chloro-4-fluoro-phényl)-{4-fluoro-4-[(2-pyridin-2-yl-éthylamino)-méthyl]-piperidin-1-yl}-méthanone mais en remplaçant le 1-(3-chloro-4-fluoro-benzoyl)-4-fluoro-pipéridine-4-carbaldéhyde par le 1-(3,4-dichloro-benzoyl)-4-fluoro-pipéridine-4-carbaldéhyde.
C₂₀H₂₂Cl₂FN₃O, 1.5C₂H₂O₄ (545.37)
F : 192-194°C
¹H RMN (DMSO d₆) δ 1.70-2.10 (m, 4H) ; 3.15 (m, 3H) ; 3.31-3.50 (m, 6H) ; 4.31 (m, 1H) ; 7.27-7.41 (m, 3H) ; 7.69-7.81 (m, 3H) ; 8.49 (d, 1H).

### ETUDE PHARMACOLOGIQUE DES PRODUITS DE L'INVENTION

Les composés de cette invention ont été comparés à la 8-[4-[4-(2-pyrimidinyl)-1-pipérazilyl]butyl]-8-azaspiro[4.5] décane-7,9-dione (Buspirone) qui est un agoniste des récepteurs 5-HT_{1A} utilisé en clinique.

### 1-Mesure de l'affinité des composés de l'inventio pour les récepteurs 5-HT_{1A}

### PROTOCOLE

L'affinité in vitro des composés de l'invention pour les récepteurs 5-HT_{1A} a été déterminée par la mesure du déplacement de la (³H)8-OH-DPAT (TRK 850 ; 160-240 Ci/mmole).

L'étude de la liaison au récepteur 5-HT_{1A} est réalisée comme décrit par Sleight et Peroutka (Naunyn-Schmiedeberg's Arch. Pharmaco. 1991, 343, 106). Pour ces expérimentations, des cortex cérébraux de rat sont utilisés. Après décongélation du cerveau dans du tampon Tris-HCI 50 mmoles, pH = 7.40 à 25°C, le cortex cérébral est prélevé et homogénéisé dans 20 volumes de tampon maintenu à 4°C. L'homogénat est centrifugé à 39000 g pendant 10 minutes, le culot de centrifugation est mis en suspension dans le même volume de tampon et centrifugé à nouveau. Après une nouvelle mise en suspension dans les mêmes conditions, l'homogénat est incubé pendant 10 minutes à 37°C puis centrifugé à nouveau. Le culot final est mis en suspension dans du tampon de réaction froid Tris-HCl 50 mmoles, pH = 7.40 à 25°C contenant 10 mmoles de pargyline, 4 mmoles de CaCl₂ et 0.10% d'acide ascorbique. La concentration finale de tissu dans le milieu d'incubation est de 10 mg/tube.

Les tubes de réaction contiennent 0.10 ml de (³H)8-OH-DPAT (0.20 mmole en final), 0.10 ml de produit à tester 6-7 concentrations et 0.80 ml de tissu. La liaison non spécifique est définie en utilisant 10 mmoles de 5-HT. Les tubes de réaction sont incubés à 23°C pendant 30 minutes puis leur contenu est rapidement filtré sous vide sur filtres Whatman GF/B, les tubes sont rincés avec 2 fois 5 ml de tampon Tris-HCl 50 mmoles, pH = 7.4 à 25°C. La radioactivité recueillie sur le filtre est analysée en scintillation liquide en ajoutant 4 ml de liquide scintillant (Emulsifier Safe, Packard). Toutes les expériences sont réalisées en triple.

### 2-Mesure de l'affinité des composés de l'invention pour les récepteurs D₂.

### PROTOCOLE

L'affinité in vitro des composés de l'invention pour les récepteurs dopaminergiques D₂, a été déterminée par la mesure du déplacement du (³H) YM-09151-2 (NET-1004 70-87 Ci/mmole). L'étude de la liaison au récepteur D₂ est réalisée comme décrit par Niznik (Naunyn-Schmiedeberg's Arch, Pharmacol. Methods, 1985, 329, 333). Pour ces expérimentations, on utilise le striatum de rat. Après décongélation du cerveau dans du tampon Tric-HCl 50 mmoles, pH = 7.40 à 25°C, le striatum est prélevé et homogénéisé dans 40 volumes de tampon maintenu à 4°C. L'homogénat est centrifugé à 20000 g pendant 10 minutes, le culot de centrifugation est mis en suspension dans le même volume de tampon et centrifugé à nouveau. Le culot final est mis en suspension dans du tampon de réaction froid Tris-HCl 50 mmoles, pH = 7.40 à 25°C contenant 120 mmoles de NaCl et 5 mmoles de KCl. La concentration finale de tissu dans le milieu d'incubation est de 2 mg/tube. Les tubes de réaction contiennent 0.20 ml de [³H]YM-09151-2 (0.05 mmole en final), 0.20 ml de produit à tester 6-7 concentrations et 1.60 ml de tissu. La liaison non spécifique est définie en utilisant 1 mmole de (+)-Butaclamol. Les tubes de réaction sont incubés à 23°C pendant 60 minutes puis leur contenu est rapidement filtré sous vide sur filtres Whatman GF/B, les tubes sont rincés 2 fois avec 5 ml de tampon Tris-HCl 50 mmoles, pH = 7.40 à 25°C. La radioactivité recueillie sur le filtre est analysée en scintillation liquide en ajoutant 4 ml de liquide scintillant (Emulsifier Safe, Packard). Toutes les expériences sont réalisées en triple. Les constantes d'inhibition (Ki) des produits de l'invention sont estimées à partir des expérimentations de déplacement en utilisant le programme de régression non-linéaire RADLIG version 4 de EBDA (Equilibrium Binding Data Analysis) (Biosoft, Cambridge, UK, Mc Pherson, 1985). Les constantes de dissociation des ligands radioactifs utilisées dans les calculs sont de 0.31 mmole pour (³H)8-OH-DPAT et de 0.036 mmole pour (³H)YM-09151-2. Les valeurs de pKi (-logKi) sont données sous forme de moyenne ± SEM d'au moins 3 expérimentations.

### 3-Evaluation de l'activité agoniste des récepteurs 5-HT_{1A} des composés de l'invention in vivo.

### PROTOCOLE

On utilise des rats mâles Sprague Dawley (ICO : OFAD [IOPS], Iffa Credo, France) pesant 160-180 g à leur arrivée et 180-200 g au début des tests. Les animaux sont placés en quarantaine de 4 à 8 jours avec un accès libre à la nourriture standardisée de laboratoire avant leur utilisation dans les expérimentations. Les animaux sont hébergés individuellement dans des cages en plastiques sur portoir, (28 cm x 21 cm x 18 cm) avec un sol grillagé (RC Iffa Credo), 24 heures avant les tests. Grâce à un distributeur automatique, de l'eau filtrée à 0.22 µm, est disponible à volonté. La zone de quarantaine et le laboratoire d'expérimentation sont climatisés (température : 22 ± 1°C ; degré hygrométrique : 55 ± 5%) et éclairés de 7 heure à 19 heure. Tous les rats sont traités suivant l'éthique des animaux de laboratoire (Guide for the Care and Use of Laboratory Animals, U.S. Department of Agriculture. Public Health Service. National Institutes of Health publication N° 85-23, Revised 1985), et le protocole (n°15) est réalisé en accord avec les recommandations du comité d'éthique local des animaux de recherche.
Les méthodes utilisées sont essentiellement identiques à celles décrites précédemment (Drug. Dev. Res. 1992, 26, 21; Eur, J. Pharmacol. 1995, 281, 219).
On observe le comportement de l'animal pendant une période de 10 minutes chacune, centrée à t60 minutes, après administration par voie orale. Quatre animaux sont observés individuellement durant la période de 10 minutes (de t55 à t65) ; les 4 rats sont observés à tour de rôle, toutes les 15 secondes, durée de l'observation 10 secondes par animal. Durant chacune de ces périodes d'observation, on note la présence (1) ou l'absence (0) de la rétraction de la lèvre inférieure (LLR) de l'animal. On considère qu'il y a rétraction de la lèvre inférieure si l'animal présente des signes ininterrompus durant au moins 3 secondes. Ce cycle est répété 10 fois durant une période de 10 minutes, ainsi, la fréquence d'un comportement peut varier de 0 à 10 pour chaque période d'observation. Chaque jour, deux animaux de chaque groupe reçoivent la même dose du même produit. Les produits sont dissous dans de l'eau distillée, ou sont mis en suspension dans une solution aqueuse de Tween 80 (2 gouttes / 10 ml d'eau distillée). Les produits sont administrés dans un volume de 10 ml / kg et les doses sont exprimées en poids de base. L'ordre d'administration des produits et des doses est randomisé.

### RESULTATS

Le tableau 1 donne, à titre d'exemple, les pKi, la sélectivité et les doses actives (ED₅₀) pour deux dérivés de l'invention par rapport à la Buspirone, choisie comme produits de référence.

**Tableau 1**

| Composé | pKi | | Sélectivité | LLR p.o. |
|---|---|---|---|---|
| | 5-HT_{1A} | D₂ | 5-HT_{1A}/D₂ | ED₅₀ (mg / Kg) |
| Exemple 1 | 9.81 | 6.18 | 4266 | 0.31 |
| Exemple 2 | 9.42 | 6.30 | 1318 | 0.31 |
| Buspirone | 7.65 | 7.49 | 1.5 | 20 |

Les résultats des essais montrent que les composés de formule (1) possèdent une affinité élevée pour les récepteurs sérotoninergiques du sous-type 5-HT_{1A} et qu'ils sont sélectifs pour ces récepteurs, comparativement au composé de référence.
Le test in vivo, pratiqué par voie orale chez le rat, montre que les composés de formule (1) exercent une activité agoniste 5-HT_{1A} centrale plus puissante que celle du composé de référence.
Il ressort donc de cette étude, que les composés de l'invention ont l'avantage de présenter non seulement une affinité et une sélectivité pour les récepteurs sérotoninergiques du sous-type 5-HT_{1A}, supérieures à celles de la Buspirone mais, également, une activité agoniste 5-HT_{1A} par voie orale plus puissante que celle de la Buspirone : l'agoniste 5-HT_{1A} usuellement utilisé en clinique.
A ce titre, les composés de l'invention sont potentiellement utiles dans le traitement des pathologies impliquant des dysfonctionnements sérotoninergiques tels que l'anxiété, la dépression, la perception de la douleur et la neurodégénerescence.

## Revendications

1. Les composés de formule générale (1) 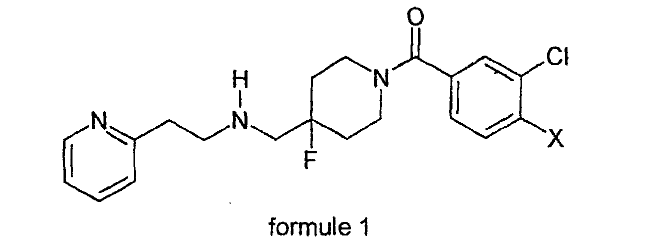 dans laquelle X représente un atome de Fluor, de chlore ou d'hydrogène,
ainsi que les sels d'addition des composés de formule générale (1) avec les acides minéraux ou les acides organiques pharmaceutiquement acceptables.

2. Le composé selon la revendication 1 : (3-Chloro-4-fluoro-phényl)-(4-fluoro-4-[(2-pyridin-2-yl-éthylamino)-méthyl]-pipéridin-1-yl}-méthanone.

3. Le composé selon la revendication 1 : (3,4-Dichlorophényl)-{4-fluoro-4-[(2-pyridin-2-yl-éthylamino)-méthyl]-pipéridin-1-yl}-méthanone.

4. Le composé selon la revendication 1 : (3-Chloro-phényl)-{4-fluoro-4-[(2-pyridin-2-yl-éthylamino)-méthyl]-pipéridin-1-yl}-méthanone.

5. Les nouveaux intermédiaires de synthèse de formule (3) 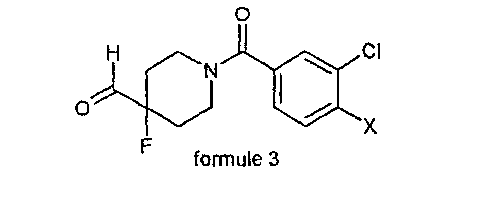 dans lesquels X est un atome de fluor, de chlore ou d'hydrogène, utilisés pour la préparation des composés de formule générale (1).

6. Utilisation d'un composé de formule (1) ou d'un sel pharmaceutique acceptable selon l'une des revendications 1 à 4, en quantité suffisante pour la préparation d'un médicament destiné au traitement de la dépression.

7. Utilisation d'un composé de formule (1) ou d'un sel pharmaceutique acceptable selon l'une des revendications 1 à 4, en quantité suffisante pour la préparation d'un médicament destiné au traitement de la perception de la douleur.

8. Utilisation d'un composé de formule (1) ou d'un sel pharmaceutique acceptable selon l'une des revendications 1 à 4, en quantité suffisante pour la préparation d'un médicament destiné au traitement de l'anxiété.

9. Utilisation d'un composé de formule (1) ou d'un sel pharmaceutique acceptable selon l'une des revendications 1 à 4, en quantité suffisante pour la préparation d'un médicament destiné au traitement de la neurodégénérescence.

10. Composition pharmaceutique **caractérisée en ce qu'**elle comprend au moins un composé de formule (1) selon l'une des revendications 1 à 4 ou un sel pharmaceutiquement acceptable d'un composé de formule (1) et un ou plusieurs excipients, adjuvants ou véhicules pharmaceutiquement acceptables appropriés.

## Claims

1. Compounds of general formula (1) 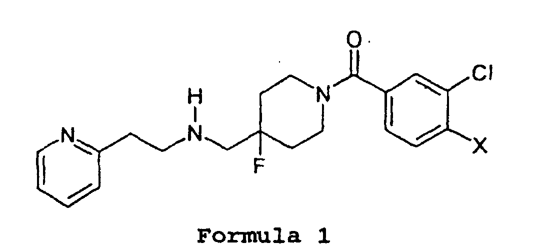 in which X represents a fluorine, chlorine or hydrogen atom,
and the addition salts of the compounds of general formula (1) with pharmaceutically acceptable mineral acids or organic acids.

2. Compound according to Claim 1: (3-chloro-4-fluorophenyl){4-fluoro-4-[(2-pyrid-2-ylethylamino)methyl]-1-piperidyl}methanone.

3. Compound according to Claim 1: (3,4-dichlorophenyl){4-fluoro-4-[(2-pyrid-2-ylethylamino)methyl]-1-piperidyl}methanone.

4. Compound according to Claim 1: (3-chlorophenyl){4-fluoro-4-[(2-pyrid-2-ylethylamino)methyl]-1-piperidyl)methanone.

5. Novel synthetic intermediates of formula (3) 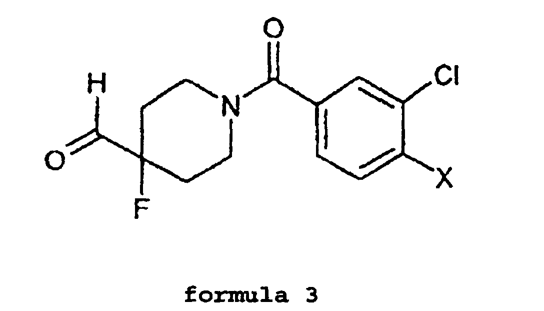 in which X is a fluorine, chlorine or hydrogen atom, which are used for the preparation of the compounds of general formula (1).

6. Use of a compound of formula (1) or of a pharmaceutically acceptable salt according to one of Claims 1 to 4, in an amount which is sufficient to prepare of a medicinal product for treating depression.

7. Use of a compound of formula (1) or of a pharmaceutically acceptable salt according to one of Claims 1 to 4, in an amount which is sufficient to prepare a medicinal product for treating the perception of pain.

8. Use of a compound of formula (1) or of a pharmaceutically acceptable salt according to one of Claims 1 to 4, in an amount which is sufficient to prepare a medicinal product for treating anxiety.

9. Use of a compound of formula (1) or of a pharmaceutically acceptable salt according to one of Claims 1 to 4, in an amount which is sufficient to prepare a medicinal product for treating neurodegeneration.

10. Pharmaceutical composition, **characterized in that** it comprises at least one compound of formula (1) according to one of Claims 1 to 4 or a pharmaceutically acceptable salt of a compound of formula (1) and one or more suitable pharmaceutically acceptable excipients, adjuvants or vehicles.

## Patentansprüche

1. Verbindungen der allgemeinen Formel (1) 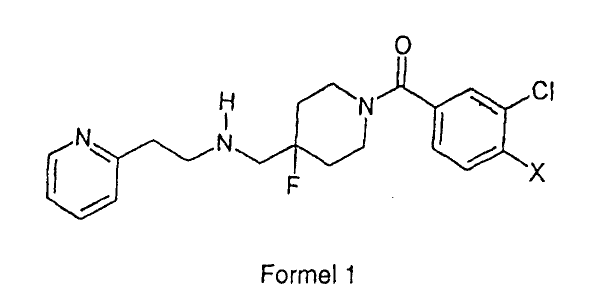 in der X ein Fluor-, Chlor- oder Wasserstoffatom darstellt,
sowie die Additionssalze der Verbindungen der allgemeinen Formel (1) mit pharmazeutisch annehmbaren Mineralsäuren oder organischen Säuren.

2. Verbindung nach Anspruch 1: (3-Chlor-4-fluorphenyl)-[4-fluor-4-[(2-pyridin-2-ylethylamino)methyl]piperidin-1-yl)methanon.

3. Verbindung nach Anspruch 1: (3,4-Dichlorphenyl)-[4-fluor-4-[(2-pyridin-2-ylethylamino)methyl]piperidin-1-yl}methanon.

4. Verbindung nach Anspruch 1: (3-Chlorphenyl)-{4-fluor-4-[(2-pyridin-2-ylethylamino)methyl]-piperidin-1-yl}methanon.

5. Neue Synthesezwischenprodukte der Formel (3) 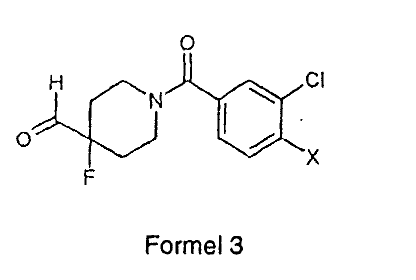 in der X ein Fluor-, Chlor- oder Wasserstoffatom ist, verwendet für die Herstellung der Verbindungen der allgemeinen Formel (1).

6. Verwendung einer Verbindung der Formel (1) oder eines pharmazeutisch annehmbaren Salzes gemäß einem der Ansprüche 1 bis 4 in ausreichender Menge für die Herstellung eines Medikamentes, das zur Behandlung von Depression bestimmt ist.

7. Verwendung einer Verbindung der Formel (1) oder eines pharmazeutisch annehmbaren Salzes gemäß einem der Ansprüche 1 bis 4 in ausreichender Menge für die Herstellung eines Medikaments, das zur Behandlung der Wahrnehmung von Schmerz bestimmt ist.

8. Verwendung einer Verbindung der Formel (1) oder eines pharmazeutisch annehmbaren Salzes nach einem der Ansprüche 1 bis 4 in ausreichender Menge für die Herstellung eines Medikaments, das zur Behandlung von Angst bestimmt ist.

9. Verwendung einer Verbindung der Formel (1) oder eines pharmazeutisch annehmbaren Salzes gemäß einem der Ansprüche 1 bis 4 in ausreichender Menge für die Herstellung eines Medikaments, das zur Behandlung von Neurodegeneration bestimmt ist.

10. Pharmazeutische Zusammensetzung, **dadurch gekennzeichnet, dass** sie mindestens eine Verbindung der Formel (1) nach einem der Ansprüche 1 bis 4 oder ein pharmazeutisch annehmbares Salz einer Verbindung der Formel (1) und ein(en) oder mehrere pharmazeutisch annehmbare geeignete Träger, Adjuvantien oder Vehikel umfasst.
